Europäisches Patentamt
European Patent Office
Office européen des brevets

(19)

(11) Veröffentlichungsnummer: **0 154 274**
**A2**

(12)

# EUROPÄISCHE PATENTANMELDUNG

(21) Anmeldenummer: 85102042.0

(22) Anmeldetag: 25.02.85

(51) Int. Cl.[4]: C 07 D 275/06
A 01 N 43/80

(30) Priorität: 08.03.84 DE 3408539

(43) Veröffentlichungstag der Anmeldung:
11.09.85 Patentblatt 85/37

(84) Benannte Vertragsstaaten:
AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Salzburg, Herbert, Dr.
Hahnenweg 1
D-5000 Köln 80(DE)

(72) Erfinder: Hajek, Manfred, Dr.
Hahnenweg 3
D-5000 Köln 80(DE)

(72) Erfinder: Hänssler, Gerd, Dr.
Heymannstrasse 40
D-5090 Leverkusen 1(DE)

(54) **Verfahren zur Herstellung von 3-Hydrazino-1,2-benzisothiazol-1,1-dioxiden.**

(57) Die Erfindung betrifft ein neues Verfahren zur Herstellung bekannter 3-Hydrazino-1,2-benzisothiazol-1,1-dioxide der Formel (I)

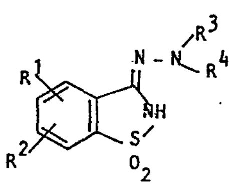

(I);

in welcher

$R^1$ und $R^2$ gleich oder verschieden sein können und für Wasserstoff, für Alkyl, für Halogen, für Amino, für Hydroxy oder für Carboxy stehen und

$R^3$ und $R^4$ gleich oder verschieden sein können und für Wasserstoff, für Alkyl, für Cycloalkyl oder für Aryl stehen, in sehr guten Ausbeuten und hoher Reinheit, indem man 3-Keto-2H, 3H-1,2-benzisothiazol-1,1-dioxide der Formel (II)

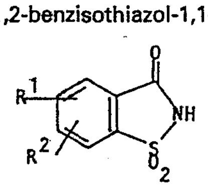

(II)

in welcher

$R^1$ und $R^2$ die oben angegebenen Bedeutungen haben mit Hydrazinen der Formel (III)

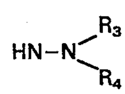

(III)

in welcher

$R^3$ und $R^4$ die oben angegebenen Bedeutungen haben, in einem Molverhältnis von mindestens 1:1 bis 1:50, in einem inerten Lösungsmittel, bei Temperaturen zwischen 75°C and 200°C und bei einer Reaktionsdauer von 15 bis 40 Stunden umsetzt sowie die Verwendung der Verbindungen der Formel (I) als Fungizide und als Zwischen-produkte zur Herstellung z.B. von Schädlings-bekämpfungsmitteln und Pharmazeutika.

EP 0 154 274 A2

Croydon Printing Company Ltd.

BAYER AKTIENGESELLSCHAFT 5090 Leverkusen, Bayerwerk
Konzernverwaltung RP
Patentabteilung Bas/Kü-c
IV/ZP/II

Verfahren zur Herstellung von 3-Hydrazino-1,2-benzisothiazol-1,1-dioxiden

Die Erfindung betrifft ein neues Verfahren zur Herstellung von bekannten 3-Hydrazino-1,2-benzisothiazol-1,1-dioxiden, die z.B. eine pharmakologische Wirkung oder eine Wirkung auf dem Pflanzenschutzgebiet aufweisen und außerdem als Zwischenprodukte für die Synthese von Wirkstoffen zur Schädlingsbekämpfung, insbesondere als Fungizide, in der Pharmakologie oder als Polymeraktivatoren verwendet werden können.
Es ist bereits bekannt, daß man 3-Hydrazino-1,2-benzisothiazol-1,1-dioxide erhält, wenn man die entsprechenden 3-Chlor oder 3-Alkoxy-Derivate mit Hydrazin oder substituierten Hydrazinen umsetzt /vgl. E. Schrader, Prakt. Chemie 95, 312-326 (1917); US-Pat. Nr. 4 178 451/.

Ein anderer Weg führt über die 3-Mercapto-Verbindung, die ihrerseits aus der 3-Chlor-Verbindung hergestellt wird, zu den gewünschten 3-Hydrazino-Verbindungen.

Die 3-Chlor-Verbindungen werden durch Chlorierung von 3-Keto-2H,3H-1,2-benzisothiazol-1,1-dioxid mit Chlorierungsmitteln, wie z.B. Phosphorpentachlorid, gewonnen, wobei eine beträchtliche Bildung von Nebenprodukten auftritt [vgl. H. Böhme u.a. Zeitschrift für Analytische Chemie 139, 255 ff (1953)].

All diesen Verfahren ist gemeinsam, daß erst die 3-Keto-2H,3H-1,2-benzisothiazol-1,1-dioxide in Zwischenprodukte überführt werden, die in 3-Stellung eine Chlor-, Alkoxy- oder Mercapto-gruppe tragen und diese dann mit gegebenenfalls substituierten Hydrazinen umgesetzt werden.

Weiterhin ist bekannt, daß man 3-Hydroxy-1,2-benzisothiazol-1,1-dioxide mit Hydrazin unter Normaldruck umsetzen kann, wobei unter Ringöffnung das o-Sulfamidobenzhydrazid entsteht. Arbeitet man mit einem Überschuß an wasserfreiem Hydrazin bei 125°C im Bombenrohr, so werden zwei Verbindungen gewonnen. Erstens erhält man 1-Amino-2,5-(2-sulfamidophenyl)-1,3,4-triazol, das durch Austritt von 2 Mol Wasser aus dem Ringöffnungsprodukt - o-Sulfamido-benzhydrazid - entstanden ist, und zweitens kann nach Ansäuren des Filtrates in geringen Ausbeuten die gewünschte 3-Hydrazino-Verbindung gewonnen werden [vgl. E. Schrader, Prakt. Chemie 95,312-326 (1917)].
Es wurde nun gefunden, daß man die 3-Hydrazino-1,2-benzisothiazol-1,1-dioxide der Formel (I)

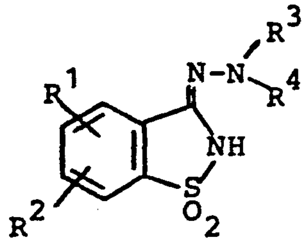

(I)

in welcher

$R^1$ und $R^2$ gleich oder verschieden sein können und für Wasserstoff, für Alkyl, für Halogen, für Amino, für Hydroxy oder für Carboxy stehen und

$R^3$ und $R^4$ gleich oder verschieden sein können und für Wasserstoff, für Alkyl, für Cycloalkyl oder für Aryl stehen,

in sehr guten Ausbeuten und hoher Reinheit erhält, wenn man 3-Keto-2H,3H-1,2-benzisothiazol-1,1-dioxide der Formel (II)

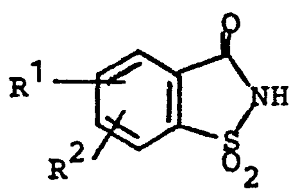

(II)

in welcher

$R^1$ und $R^2$ die oben angegebenen Bedeutungen haben, mit Hydrazinen der Formel (III)

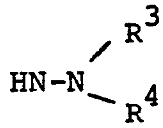

(III)

in welcher

$R^3$ und $R^4$ die oben gegebene Bedeutung haben, in einem Molverhältnis von mindestens 1:1 bis 1:50,

in einem inerten Lösungsmittel, bei Temperaturen zwischen 75°C und 200°C und bei Reaktionszeiten von 15 bis 40 Stunden umsetzt.

Die Verbindungen der Formel (I) liegen im allgemeinen im Gleichgewicht mit den Verbindungen der Formel (IA) vor:

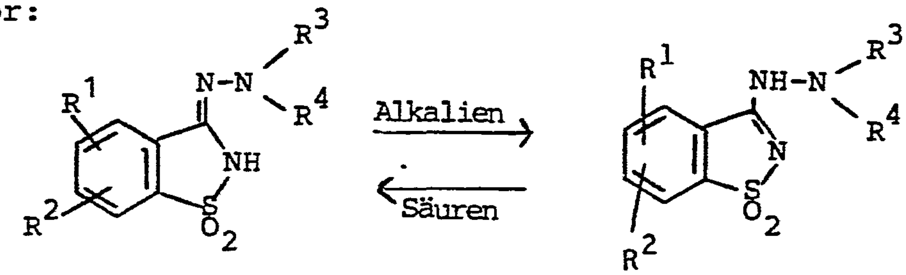


(I) (IA)

Im nachfolgenden wird der Einfachheit halber stets von Verbindungen der Formel (I) gesprochen, obwohl die reinen Verbindungen oder ihre Gemische mit verschiedenen Anteilen der Verbindungen der Formel (I) und (IA) gemeint sind.

Es ist als ausgesprochen überraschend zu bezeichnen, daß gemäß der erfindungsgemäßen Umsetzung von 3-Keto-2H,3H-1,2-benzisothiazol-1,1-dioxiden der Formel (II)

mit Hydrazinen der Formel (III) die gewünschten Verbindungen in so hoher Ausbeute und Reinheit erhalten werden, weil man im Hinblick auf den Stand der Technik erwarten mußte, daß die Reaktion unter Ringöffnung erfolgt und Gemische der verschiedensten Verbindungen entstehen.

Das erfindungsgemäße Verfahren weist eine Reihe von Vorteilen auf. So erhält man die 3-Hydrazino-Verbindungen der Formel (I) in einem Einstufenverfahren in hohen Ausbeuten und hoher Reinheit. Die benötigten Ausgangsstoffe sind käuflich zu erwerben oder nach bekannten Verfahren und in guten Ausbeuten leicht zugänglich.

Verwendet man 6-Chlor-3-keto-2H,3H-1,2-benzisothiazol-1,1-dioxid und Hydrazin-Hydrat als Ausgangsstoffe, so kann der Reaktionsablauf durch das folgende Formelschema wiedergegeben werden:

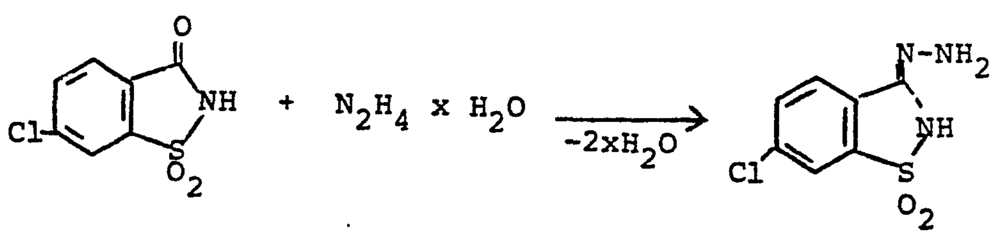


Die als Ausgangsverbindungen einzusetzenden 3-Keto-2H,3H-1,2-benzisothiazol-1,1-dioxide sind durch die Formel (II) allgemein definiert. Darin sind

$R^1$ und $R^2$ gleich oder verschieden und stehen für Wasserstoff, für Alkyl mit 1 bis 5 Kohlenstoffatomen, für Fluor, Chlor, Brom, für Amino, für Hydroxy oder für Carboxy.

Die erfindungsgemäß verwendbaren 3-Keto-2H,3H-1,2-benzisothiazol-1,1-dioxide sind bereits bekannt und nach allgemein bekannten Verfahren herstellbar. Als Beispiele seien z.B. genannt:
3-Keto-2H,3H-1,2-benzisothiazol-1,1-dioxid,
5-Chlor-, 5-Brom-, 5-Methyl-, 5-Ethyl-, 5,6-Dichlor-, 6-Chlor-, 6-Brom-, 6-Methyl-, 6-Ethyl-, 5-Amino-, 5-Hydroxy- oder 5-Carboxy-3-keto-2H,3H-1,2-benzisothiazol-1,1-dioxid.

Das weiterhin als Ausgangsverbindung einzusetzende Hydrazin oder seine Substitutionsprodukte sind durch die Formel (III) allgemein definiert.

$R^3$ und $R^4$ sind gleich oder verschieden und stehen für Wasserstoff, für Alkyl mit 1 bis 6 Kohlenstoffatomen, wie Methyl, Ethyl, n- und iso-Propyl, n-, sec.-, tert.- und iso-Butyl, n-Pentyl, tert.-Pentyl und Hexyl, für Cycloalkyl mit 3 bis 6 Kohlenstoffatomen, wie Cyclopropyl, Cyclopentyl und Cyclohexyl, oder für Phenyl.

Die erfindungsgemäß verwendbaren Verbindungen der Formel (III) sind bekannte Verbindungen und nach allgemein bekannten Verfahren herstellbar. Als Beispiele

seien z.B. genannt: Hydrazin, Methylhydrazin, Ethylhydrazin, n-Propylhydrazin, iso-Propylhydrazin, tert.-Butylhydrazin, n-Hexylhydrazin, N,N-Dimethylhydrazin, N,N-Diethylhydrazin, Cyclopropylhydrazin, Cyclohexylhydrazin und Phenylhydrazin.

Als Lösungsmittel kommen für das erfindungsgemäße Verfahren alle inerten organischen Lösungsmittel infrage. Hierzu gehören Alkohole, wie z.B. Ethanol, Propanol, Butanole, Pentanole und Cyclohexylalkohol, insbesondere n- und sek.-Butanol und Pentanol, aromatische Kohlenwasserstoffe, wie Chlorbenzol, Dichlorbenzol, chlorierte Xylole; ferner Carbonsäuren, wie Essigsäure und Propionsäure. Auch Gemische aus diesen Lösungsmitteln können verwendet werden.

Die erfindungsgemäße Umsetzung wird bei Temperaturen zwischen 75°C und 200°C, vorzugsweise zwischen 130°C und 165°C vorgenommen.

Die Umsetzung wird bei Normaldruck ausgeführt.

Bei der Durchführung des erfindungsgemäßen Verfahrens setzt man die Ausgangsverbindungen in einem Molverhältnis von mindestens 1:1 bis zu einem 50-fachen Hydrazin-Komponentenüberschuß ein. Bevorzugt setzt man die Hydrazin-Komponente im 5- bis 10-fachen, insbesondere im 7-fachen Überschuß ein.

Bei der erfindungsgemäßen Umsetzung beträgt die Reaktionsdauer 15 bis 40 Stunden, vorzugsweise 30 bis 35 Stunden.

Die nach dem erfindungsgemäßen Verfahren herstellbaren Verbindungen sind bekannt, ebenso wie ihre Wirkungen z.B. in der Pharmakologie /¯vgl. Whitehead u.a., J. Med. Chem. 10, 844 (1967)_7. Sie können aber auch als Zwischenprodukte z.B. zur Herstellung für Pflanzenschutzmittel, Polymeraktivatoren und Pharmazeutika verwendet werden.

So kann man die Verbindungen der Formel (I), in denen $R^3$ und $R^4$ für Wasserstoff stehen, mit Aldehyden oder Ketonen unter üblichen Bedingungen z.B. zu Hydrazonen umsetzen (vgl. Organikem, VEB Deutscher Verlag der Wissenschaften, Berlin 1977). Diese Verbindungen weisen pharmakologische Eigenschaften auf /¯vgl. Whitehead u.a., J. Med. Chem. 10, 844 (1967)_7. Die Wirkung dieser Folgeprodukte auf dem Pflanzenschutzgebiet ist neu und Gegenstand einer noch nicht veröffentlichten Anmeldung; ebenso ist die Verwendung der nach dem erfindungsgemäßen Verfahren herzustellenden Verbindungen auf dem Pflanzenschutzgebiet neu, nicht nur die Verwendung der Umsetzungsprodukte.

Die nach dem erfindungsgemäßen Verfahren hergestellten Stoffe und deren Folgeprodukte weisen eine starke mikrobizide Wirkung auf und können zur Bekämpfung von unerwünschten Mikroorganismen praktisch eingesetzt werden. Sie sind für den Gebrauch als Pflanzenschutzmittel, insbesondere als Fungizide und Bakterizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Stoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die Stoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe,

wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

0154274

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die nach dem erfindungsgemäßen Verfahren hergestellten Stoffe und deren Folgeprodukte können in den Formulierungen in Mischung mit anderen bekannten Wirkstoffen vorliegen, wie andere Fungizide, Insektizide, Akarizide sowie in Mischungen mit Düngemitteln und Wachstumsregulatoren.

Die Stoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten,

lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

0154274

Anwendungsbeispiele:

In den folgenden Anwendungsbeispielen wird die hier angegebene Verbindung als Vergleichssubstanz herangezogen.

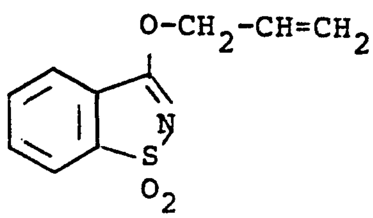

(A)

Beispiel A

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigt in diesem Test z.B. das folgende Beispiel:

Tabelle A

Pyricularia-Test (Reis) / protektiv

| Wirkstoffe | Wirkstoff-konzentration in % | Krankheitsbefall in % der unbehandelten Kontrolle |
|---|---|---|
| 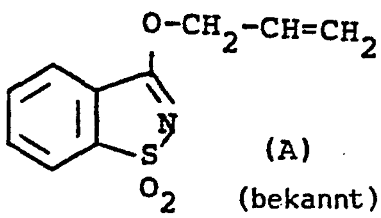 (A) (bekannt) | 0,025 | 25 |
| 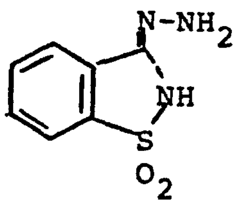 | 0,025 | 0 |

Beispiel B

Pyricularia-Test (Reis) /systemisch

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100 % bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die folgenden Beispiele :

T a b e l l e B

Pyricularia-Test (Reis) / systemisch

| Wirkstoffe | Aufwandmenge in mg Wirkstoff pro 100 cm² | Krankheitsbefall in % der unbehandelten Kontrolle |
|---|---|---|
| 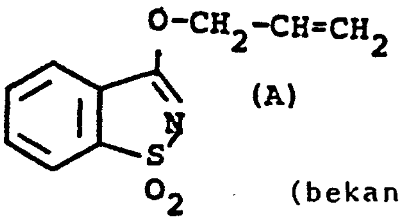 (A) (bekannt) | 100 | 50 |
| 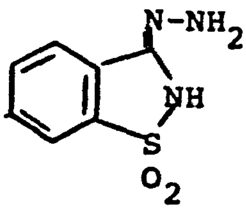 | 100 | 30 |
| 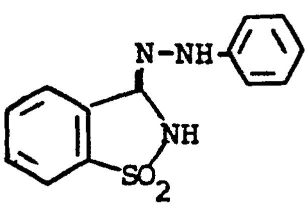 | 100 | 11 |

Die nach dem erfindungsgemäßen Verfahren zugänglichen Verbindungen sind z.B. 3-Hydrazino-1,2-benzisothiazol-1,1-dioxid, 3-(N,N-Dimethylhydrazino)-1,2-benzisothiazol-1,1-dioxid, 3-t-Butylhydrazino-1,2-benzisothiazol-1,1-dioxid, 3-(N-Ethyl-N-methylhydrazino)-1,2-benzisothiazol-1,1-dioxid, 5-Chlor-3-hydrazino-1,2-benzisothiazol-1,1-dioxid, 6-Chlor-3-hydrazino-1,2-benzisothiazol-1,1-dioxid, 5,6-Dichlor-3-hydrazino-1,2-benzisothiazol-1,1-dioxid, 5-Chlor-6-methyl-3-hydrazino-1,2-benzisothiazol-1,1-dioxid.

Die nachfolgenden Herstellungsbeispiele sollen die Erfindung näher erläutern, ohne sie zu beschränken.

Herstellungsbeispiele

Beispiel 1

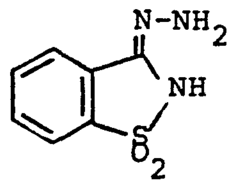

36,6 g (0,2 Mol) 3-Keto-2H,3H-1,2-benzisothiazol -1,1-dioxid und 20 g Hydrazinhydrat werden in 200 ml n-Butanol ca. 35 h am Rückfluß gekocht. Anschließend wird die gelbe Lösung im Vakuum eingeengt und mit 50 ml Essigsäure verrührt. Der Kristallbrei wird abgesaugt und das so gewonnene Rohprodukt mit dem Schmelzpunkt von 249°C kann aus Wasser umkristallisiert werden. Man erhält 36,3 g (91 % der Theorie) 3-Hydrazino-2H,3H-1,2-benzisothiazol-1,1-dioxid mit dem Schmelzpunkt von 263°C.

Beispiel 2

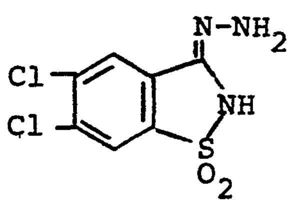

50,2 g (0,2 Mol) 5,6-Dichlor-3-keto-2H,3H-1,2-benzisothiazol-1,1-dioxid werden in 200 ml Diethylenglykol mit 35 g Hydrazinhydrat 30 h bei 160°C bis 165°C gerührt. Nach Aufarbeitung analog Beispiel 1 erhält man

31,7 g (63 % der Theorie) 5,6-Dichlor-3-hydrazino-1,2-benzisothiazol-1,1-dioxid mit dem Zersetzungspunkt von 276°C.

Beispiel 3

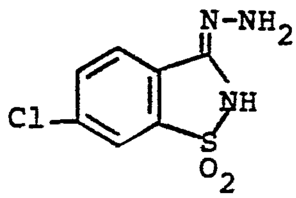

6-Chlor-3-hydrazino-1,2-benzisothiazol-1,1-dioxid erhält man analog Beispiel 1 durch Umsetzung von 6-Chlor-3-keto-2H,3H-1,2-benzisothiazol-1,1-dioxid (43,2 g; 0,2 Mol) mit 35 g Hydrazinhydrat in n-Pentanol bei 150°C. Ausbeute 33,7 g (78 % der Theorie). Fp.= 277°C.

Beispiel 4

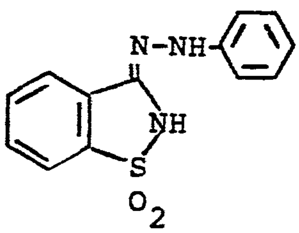

3-Phenyl-hydrazino-1,2-benzisothiazol-1,1-dioxid erhält man analog Beispiel 1 aus 3-Keto-2H,3H-1,2-benzisothiazol-1,1-dioxid und Phenylhydrazin in 74 % Ausbeute mit dem Zersetzungspunkt 225°C.

Beispiel 5

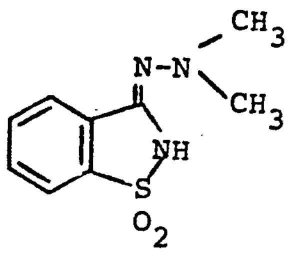

3-(N,N-Dimethylhydrazino)-1,2-benzisothiazol-1,1-dioxid erhält man analog Beispiel 1 aus 3-Keto-2H,3H-1,2-benzisothiazol-1,1-dioxid und N,N-Dimethylhydrazin in 69 % Ausbeute mit dem Zersetzungsprodukt 238°C.

Beispiel 6

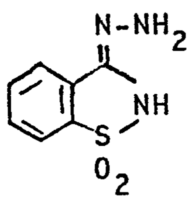

36,6 g (0,2 Mol) 3-Keto-2H,3H-1,2 benzisothiazol-1,1-dioxid und 70 g (1,4 Mol) Hydrazinhydrat werden in 250 ml n-Butanol ca. 25 h am Rückfluß gekocht. Anschließend wird mit weiteren 250 ml n-Butanol verdünnt und im Vakuum eingeengt. Der halbkristalline Rückstand wird mit 50 ml Eisessig verrührt und die entstandene Kirstall-Suspension abgesaugt. Man erhält 37,2 g (94% der Theorie) 3-Hydrazino-2H,3H-1,2-benzisothiazol-1,1-dioxid mit einem Schmelzpunkt von 262° C.

Patentansprüche

1. Verfahren zur Herstellung von 3-Hydrazino-1,2-benzisothiazol-1,1-dioxiden der Formel (I)

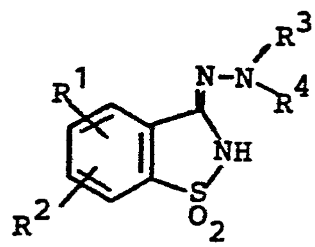

(I)

in welcher

$R^1$ und $R^2$ gleich oder verschieden sein können und für Wasserstoff, für Alkyl, für Halogen, für Amino, für Hydroxy oder für Carboxy stehen und

$R^3$ und $R^4$ gleich oder verschieden sein können und für Wasserstoff, für Alkyl, für Cycloalkyl oder für Aryl stehen,

dadurch gekennzeichnet, daß man 3-Keto-2H,3H-1,2-benzisothiazol-1,1-dioxide der Formel (II)

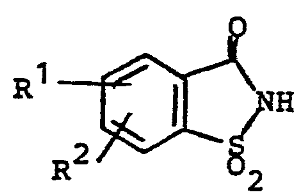

(II)

in welcher

$R^1$ und $R^2$ die oben angegebenen Bedeutungen haben, mit Hydrazinen der Formel (III)

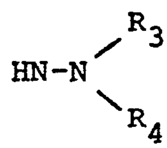

(III)

in welcher

$R^3$ und $R^4$ die oben angegebenen Bedeutungen haben, in einem Molverhältnis von mindestens 1:1 bis 1:50, in einem inerten Lösungsmittel, bei Temperaturen zwischen 75°C und 200°C und bei einer Reaktionsdauer von 15 bis 40 Stunden umsetzt.

2. Verfahren gemäß Anspruch 1, dadurch gekennzeichnet, daß das Molverhältnis 1:5 bis 1:10 beträgt.

3. Verfahren gemäß Anspruch 1, dadurch gekennzeichnet, daß die Temperaturen zwischen 130°C und 165°C liegen.

4. Verfahren gemäß Anspruch 1, dadurch gekennzeichnet, daß Butanole und Pentanole als Lösungsmittel verwendet werden.

5. Verfahren gemäß Anspruch 1, dadurch gekennzeichnet, daß die Reaktionsdauer zwischen 30 und 35 Stunden liegt.

6. Verwendung von 3-Hydrazino-1,2-benzisothiazol-1,1-dioxiden der Formel (I) zur Bekämpfung von Pilzen.

7. Fungizide Mittel, gekennzeichnet durch einen Gehalt an mindestens einem 3-Hydrazino-1,2-benzisothiazol-1,1-dioxid der Formel (I).

8. Verfahren zur Bekämpfung von Pilzen, dadurch gekennzeichnet, daß man 3-Hydrazino-1,2-benzisothiazol-1,1-dioxide der Formel (I) auf Pilze oder ihren Lebensraum einwirken läßt.

9. Verfahren zur Herstellung von fungiziden Mitteln, dadurch gekennzeichnet, daß man 3-Hydrazino-1,2-benzisothiazol-1,1-dioxide der Formel (I) mit Streckmitteln und/oder oberflächenaktiven Mitteln vermischt.